# NEUE EUROPÄISCHE PATENTSCHRIFT

(11) **EP 0 252 406 B2**
(45) Veröffentlichungstag und Bekanntmachung des Hinweises auf die Entscheidung über den Einspruch: **20.03.1996**
(45) Hinweis auf die Patenterteilung: 31.03.1993
(21) Anmeldenummer: 87109361.3
(22) Anmeldetag: 30.06.1987
(51) Int. Cl.: C09B 57/00, C07D 493/04, C07D 495/04, C07D 487/04

(54) **Verfahren zur Herstellung polycyclischer Verbindungen**
Process for the manufacture of polycyclic compounds
Procédé de fabrication de composés polycycliques

(30) Priorität: 10.07.1986 DE 3623156
(43) Veröffentlichungstag der Anmeldung: 13.01.1988
(73) Patentinhaber: BAYER AG, D-51368 Leverkusen (DE)
(72) Erfinder: Fürstenwerth, Hauke, Dr., D-5090 Leverkusen 3 (DE)

(56) Entgegenhaltungen:
- EP-A- 0 033 583
- DE-A- 3 525 109
- FR-A- 2 343 785
- GB-A- 2 103 231
- American Dyestuff Reporter, 3, 37 (1970). C Muller
- Ullmann's Encyklopädie der techn. Chemie, 4. Auflage, Bd.13 (1977) Seiten 153, 156, 157
- The Chemistry of the Quinonoid Compounds, part 2 (1974) Seiten 1047-1072. Th Finley.
- Monatshefte für Chemie 99, 2359 (1988), H Junek et al.

## Beschreibung

Gegenstand der Erfindung ist ein neues Verfahren zur Herstellung von Verbindungen der Formel 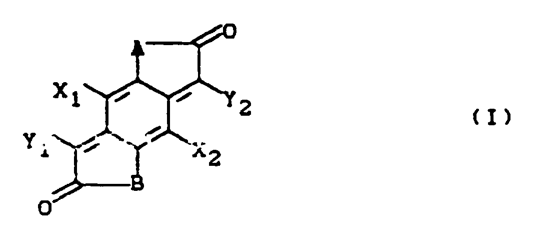 worin
A und B O, S oder NQ,
- X₁ und X₂: Wasserstoff, Halogen, Alkyl, Alkoxy, Aryl oder Z und
- Y₁ und Y₂: Alkyl, Cycloalkyl, Aryl oder Hetaryl bedeuten, wobei die genannten Reste gleich oder verschieden sind und
- Q: für V oder Acyl
- Z: für COOV, CN oder CONVW
- V: für Wasserstoff, Alkyl oder Aryl und
- W: für V stehen.

Die vorstehend genannten Reste können durch in der Farbstoffchemie übliche Substituenten weiter substituiert sein.

Die Verbindungen der Formel 1, in denen Y₁ und Y₂ Arylrestebedeuten, sind bekannte wertvolle Farbstoffe. Sie wurden bislang z.B. in der Weise hergestellt, daß man Mandelsäurederivate entweder mit Verbindungen oder Formel lla oder llb 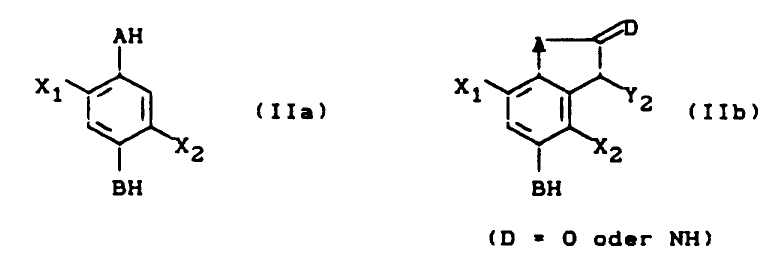 bei erhöhten Temperaturen umsetzt und das Umsetzungsprodukt anschließend oxidiert (vgl. EP-A 33 583 und US-A 4 115 404).

Es wurde nun überraschenderweise gefunden, daß man die Farbstoffe der Formel 1 wesentlich einfacher - nämlich in einer Einstufenreaktion und unter deutlich milderen Bedingungen erhält, wenn man die Verbindungen die in einer ihrer tautomeren Formen der Formel llb entsprechen mit α-Ketosäurederivaten der Formel 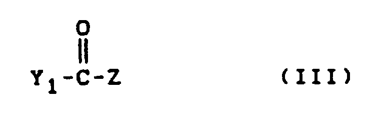 wobei Y₁ und Z die obengenannte Bedeutung haben, mit sauren Kondensationsmitteln umsetzt.

Diese Kondensation kann sowohl in der Schmelze als auch in Gegenwart eines geeigneten Lösungsmittels, das gleichzeitig als Kondensationsmittel dienen kann, durchgeführt werden.

Als Lösungsmittel können eingesetzt werden: Alkohole. aromatische Kohlenwasserstoffe. Chlorbenzole, Nitrobenzol, Carbonsäuren, Glykolether u.s.w..

Geeignete Kondensationsmittel sind Proton- und Lewissäuren, wie z.B. Mineralsäuren, aliphatische und aromatische Sulfonsäuren, BF₃, ZnCl₂, FeCl₃, AlCl₃, saure lonenaustauscher.

Die Reaktionstemperaturen können in einem größeren Bereich variiert werden. Vorzugsweise arbeitet man bei 50 bis 120°C, insbesondere bei 80 - 100° C.

Die Ausgangsmaterialien der Formeln IIb und III werden im allgemeinen in äquimolaren Mengen eingesetzt. Zur Erzielung optimaler Ausbeuten kann aber im Einzelfall die Verwendung eines geringen Überschusses empfehlenswert sein.

Die Ketosäuren de Formel III sind allgemein bekannt bzw. nach herkömmlichen Methoden leicht zugänglich.

Auch die Verbindungen der Formel llb mit D=0 sind in der Literatur beschrieben (vgl. GB-A 2 103 231). Man erhält diese Verbindungen beispielsweise durch saure Kondensation von Verbindungen der Formel lla mit Mandelsäurederivaten der Formel 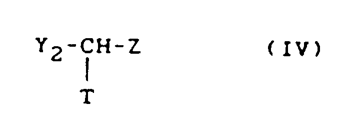 worin
- Y₂ und Z: die obengenannte Bedeutung haben und
- Z: für OH, O-Acyl oder Halogen steht.

Verbindungen der Formel llb mit D=NH werden durch Addition von methylenaktiven Nitrilen an Benzochinone hergestellt.

Gemäß einer besonders zweckmäßigen Variante des erfindungsgemäßen Verfahrens werden die Farbstoffe der Formel I in einer Eintopfreaktion dadurch hergestellt, daß man die Verbindungen der Formeln lla und IV zu den Verbindungen der Formel IIb in Gegenwart saurer Kondensationsmittel umsetzt und anschließend die Verbindung IIb ohne deren Zwischenisolierung mit den Ketosäurederivaten der Formel III zu Ende kondensiert.

Die Reaktionsbedingungen entsprechen dabei den Verhältnissen bei der Einzelsynthese der entsprechenden Verbindungen.

In den obengenannten Formeln haben die dort genannten Reste folgende bevorzugte Bedeutungen:
- Acyl =: Alkylcarbonyl oder Arylcarbonyl.
- Alkyl =: C₁-C₆-Alkyl, das gesättigt oder ungesättigt ist und gegebenenfalls durch Phenyl, Halogen, OH, CN, COOH, COOC₁-C₄-Alkyl oder O-C₁-C₄-Alkyl substituiert sein kann,
- Halogen =: F, Br und vor allem Cl,
- Cycloalkyl =: Cyclohexyl
- Aryl =: Phenyl oder Naphthyl, das durch Halogen. C₁-C₄-Alkyl oder C₁-C₄-Alkoxy substituiert sein kann,
- Hetaryl =: Thiophen-, Furan-, Pyrazol-, Triazol-, Thiazol-, Benzthiazol-, Benzoxazol- oder Benzimidazolreste.

Bevorzugte Verfahrensprodukte sind solche der Formel I,
worin
- A und B =: O,
- X₁ und X₂ =: H und
- Y₁ und Y₂ =: Aryl (in seiner allgemeinen und bevorzugten Bedeutung) sind Bevorzugte Verbindungen der Formeln III und IV sind die entsprechenden Alkylester. In den nachfolgenden Beispielen bedeuten "Teile" Gewichtsteile.

### Beispiel 1

22,6 Teile der Verbindung 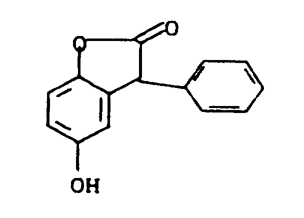 werden mit 17 Teilen Phenylglyoxylsäuremethylester, 150 Teilen 73 %iger Schwefelsäure eine Stunde bei 120° C gerührt. Danach wird mit 100 Teilen Wasser verdünnt, abgekühlt und die ausgefallenen Kristalle werden isoliert, mit Wasser und Methanol gewaschen. Nach dem Trocknen erhält man 25 Teile Farbstoff der Formel 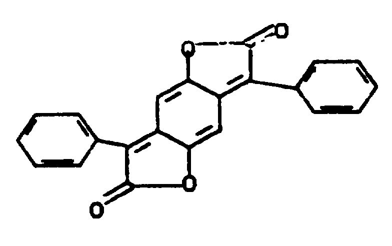 welcher in allen seinen Eigenschaften identisch ist mit dem nach den Angaben der GB-A 2 103 231 hergestellten Produkt.

### Beispiel 2

11,3 Teile 5-Hydroxy-2-oxo-3-phenyl-2,3-dihydrobenzofuran und 8,5 Teile Phenylglyoxylsäuremethylester werden in einer Mischung aus 50 Teilen Eisessig und 5 Teilen Schwefelsäure 4 Stunden bei ca. 115° C am Rückfluß gekocht. Danach wird mit 100 Teilen Methanol verdünnt. Nach Abkühlen, Isolierung und Trocknung werden 10 Teile Farbstoff erhalten, welcher identisch ist mit dem nach Beispiel 1 hergestellten Produkt.

### Beispiel 3

11,3 Teile 5-Hydroxy-2-oxo-3-phenyl-2,3-dihydrobenzofuran und 8,5 Teile Phenylglyoxylsäuremethylester werden in 100 Teilen Toluol mit 5 Teilen para Toluolsulfonsäure 8 Stunden am Wasserabscheider unter Rückfluß erhitzt. Nach Abkühlen, isolieren und trocknen erhält man 5 Teile Farbstoff, welcher identisch ist mit dem nach Beispiel 1 hergestellten Produkt.

### Beispiel 4

24 Teile Hydrochinon werden in 150 Teilen 73 %iger Schwefelsäure mit 16 Teilen Mandelsäure eine Stunde bei 80° C gerührt. Sodann werden 25 Teile Phenylglyoxylsäuremethylester zugegeben und das Reaktionsgemisch 2 Stunden bei 120°C gerührt. Nach Aufarbeitung analog Beispiel 1 werden 25 Teile Farbstoff erhalten, der mit dem nach Beispiel 1 hergestellten Produkt identisch ist.

### Beispiel 5

11,3 Teile 5-Hydroxy-2-oxo-3-phenyl-2,3-dihydrobenzofuran werden mit 12 Teilen 4-n-propoxyphenylglyoxylsäuremethylester in 200 Teilen 73 %iger Schwefelsäure 2 Stunden bei 100°C gerührt. Nach dem Aufarbeiten erhält man 15 Teile Farbstoff der Formel

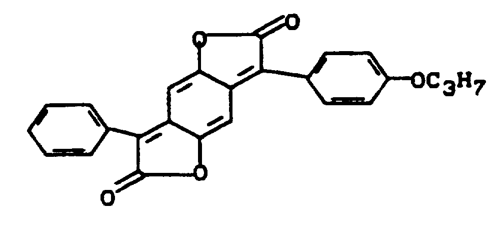

### Beispiel 6

24 Teile Hydrochinon werden in 150 Teilen 73 %iger Schwefelsäure mit 21 Teilen 4-n-propoxymandelsäure eine Stunde bei 80°C gerührt. Anschließend werden 25 Teile Phenylglyoxylsäuremethylester zugegeben und das Reaktionsgemisch 2 Stunden bei 120° C gerührt. Nach Aufarbeitung werden 28 Teile Farbstoff erhalten, der mit dem nach Beispiel 5 hergestellten Produkt identisch ist. Zum identischen Produkt gelangt man, wenn anstelle der 4-n-propoxymandelsäure 4-n-propoxymandelsäurenitril eingesetzt wird.

### Beispiel 7 - 24

Verfährt man nach den Angaben des Beispiels 6 und setzt anstelle der 4-n-propoxymandelsäure die in der folgenden Tabelle aufgeführten Mandelsäuren ein, so werden die ebenfalls in der Tabelle aufgeführten Farbstoffe erhalten.

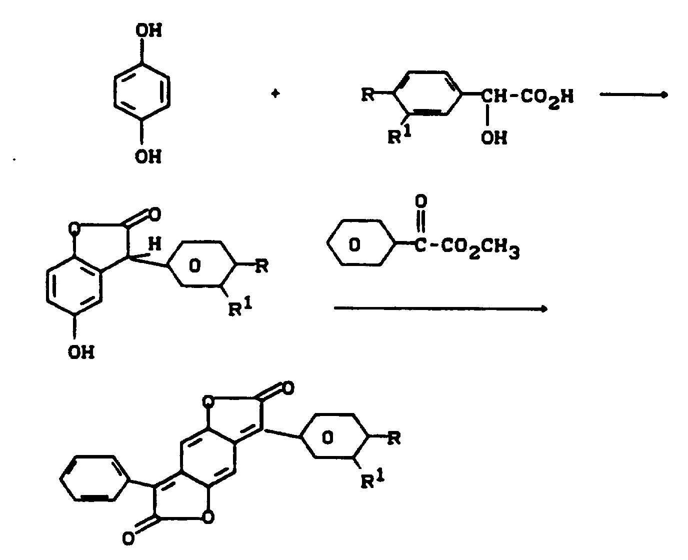

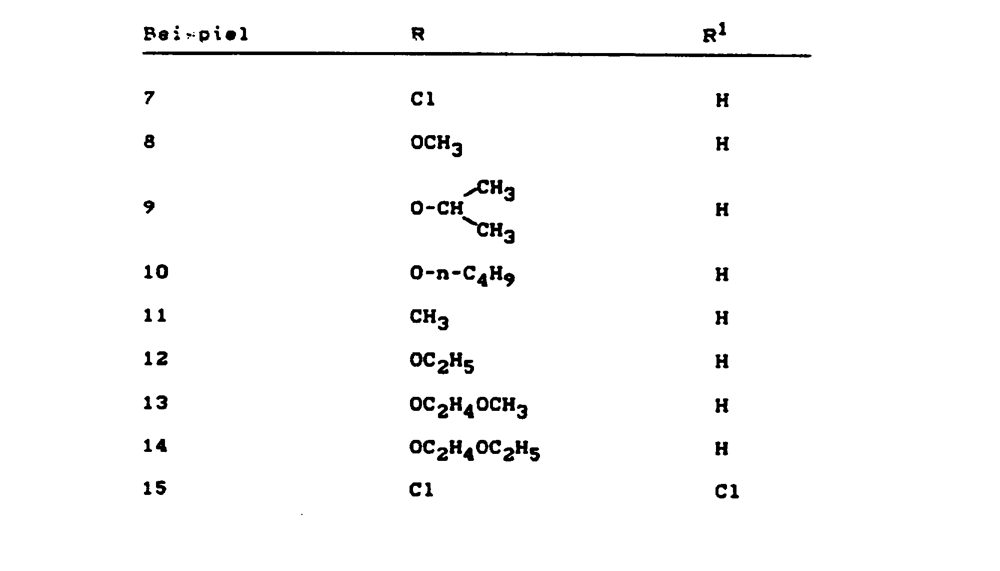

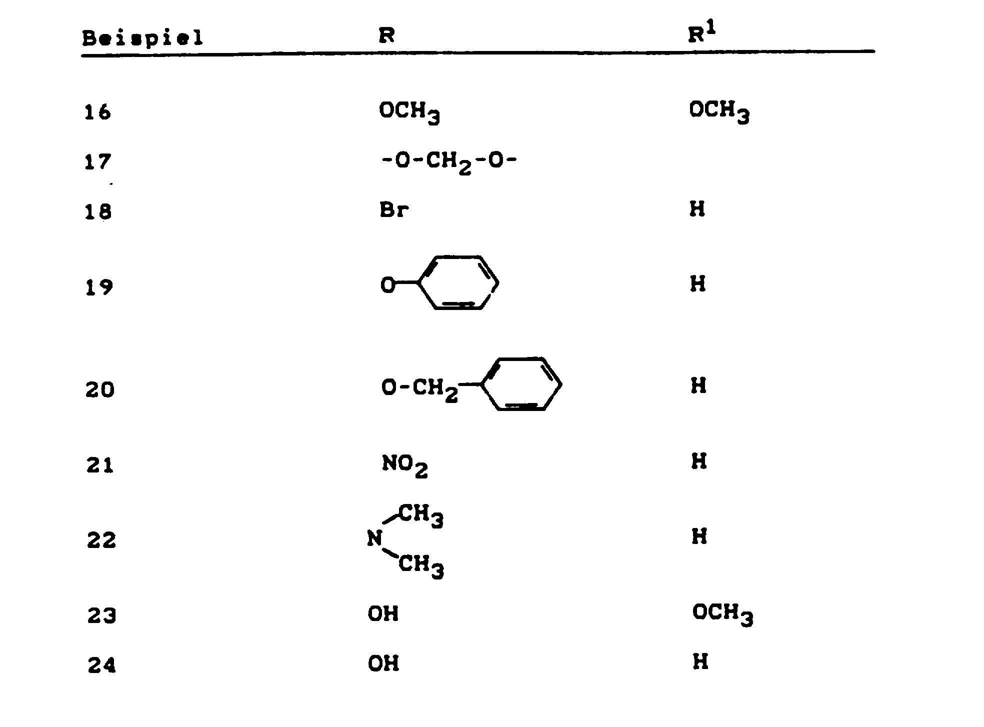

### Beispiel 25-30

Verfährt man nach den Angaben des Beispiels 4 und setzt anstelle von Phenylglyoxylsäuremethylester die in folgender Tabelle aufgeführten substituierten Phenylglyoxylester, bzw. deren Nitrile ein, so gelangt man mit vergleichbaren Ausbeuten zu den ebenfalls in der Tabelle aufgeführten Farbstoffen.

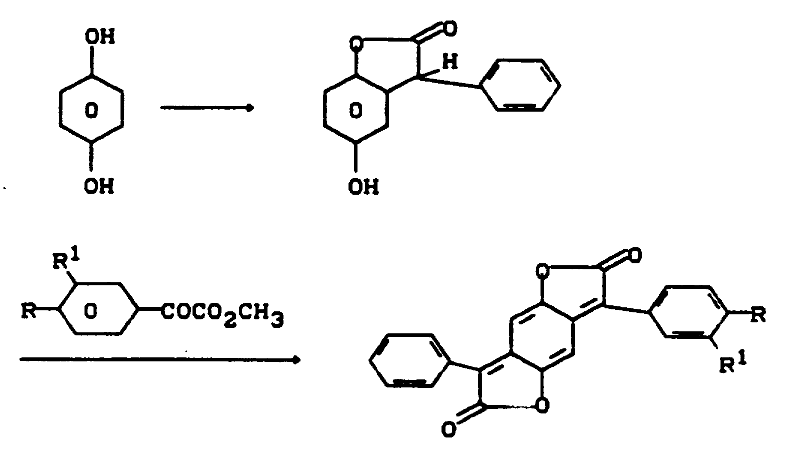

| Beispiel | R | R¹ |
|---|---|---|
| 25 | OCH₃ | CH₃ |
| 26 | OC₃H₇ | CH₃ |
| 27 | CH₃ | CH₃ |
| 28 | -OC₂H₄O- | |
| 29 | H | Cl |
| 30 | CH₃ | H |

### Beispiel 31-36

Verfährt man nach den Angaben des Beispiels 4 und setzt anstelle der Mandelsäure substituierte Mandelsäuren, bzw. deren Nitrile und anstelle des Phenylglyoxylsäuremethylesters substituierte Phenylglyoxylsäureester, bzw. deren Nitrile ein, so gelangt man zu den in der folgenden Tabelle aufgeführten Farbstoffen.

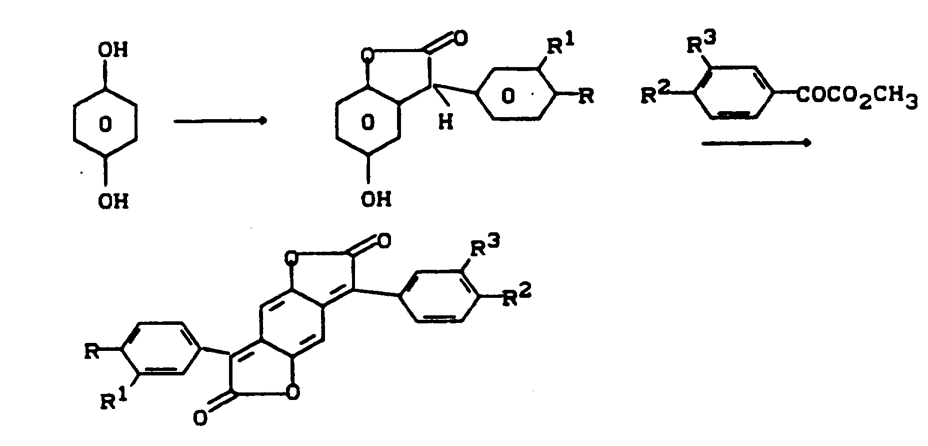

| Beispiel | R | R¹ | R | R³ |
|---|---|---|---|---|
| 31 | OCH₃ | H | OCH₃ | H |
| 32 | Cl | H | O-n-C₃H₇ | H |
| 33 | CH₃ | H | O-C₃H₅ | H |
| 34 | Cl | Cl | O-C₂H₄OCH₃ | H |
| 35 | Cl | Cl | Cl | Cl |
| 36 | O-C₃H₅ | H | O-i-C₃H₇ | H |

### Beispiel 37

22,6 Teile 5-Hydroxy-2-oxo-3-phenyl-2,3-dihydrobenzofuran und 14 Teile Methylglyoxylsäure (Brenztraubensäure) werden in 100 Teilen 73 %iger Schwefelsäure eine Stunde bei 120° C gerührt. Nach Aufarbeiten, wie in Beispiel 1 beschrieben, werden 28 Teile Farbstoff der Formel 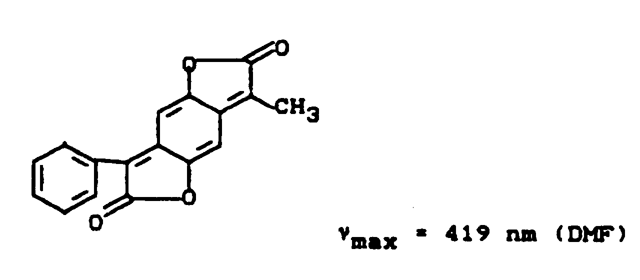 erhalten, der Polyesterfasern grünstichig gelb mit sehr guten Allgemein-Echtheiten färbt.

Auf analoger Weise lassen sich die in folgender Tabelle aufgeführten Farbstoffe ähnlicher Nuance herstellen

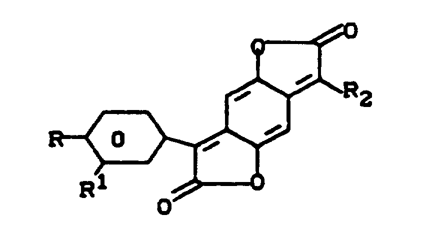

| Beispiel | R | R₁ | R₂ |
|---|---|---|---|
| 38 | OC₂H₅ | H | C(CH₃)₃ |
| 39 | H | H | C(CH₃)₃ |

| Beispiel | R | R₁ | R₂ |
|---|---|---|---|
| 40 | OC₃H₇ | H | CH₃ |
| 41 | H | H | C(CH₃)₃ |
| 42 | H | H | cyclo-C₆H₁₁ |
| 43 | OCH₃ | H | CH₂CO₂C₂H₅ |
| 44 | CH₃ | H | C(CH₃)₃ |
| 45 | Cl | Cl | C(CH₃)₃ |

## Patentansprüche

1. Verfahren zur Herstellung von Verbindungen der Formel 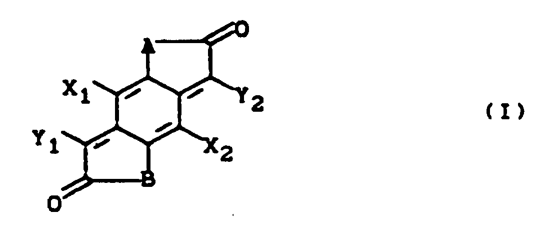 worin
A und B O, S oder NQ,
X₁ und X₂ Wasserstoff, Halogen, Alkyl, Alkoxy Aryl oder Z und
Y₁ und Y₂ Alkyl, Cycloalkyl, Aryl oder Hetaryl bedeuten, wobei die genannten Reste gleich oder verschieden sind und
Q für V oder Acyl
Z für COOV, CN oder CONVW,
V für Wasserstoff, Alkyl oder Aryl und
W für V stehen,
dadurch gekennzeichnet, daß man Verbindungen der Formel 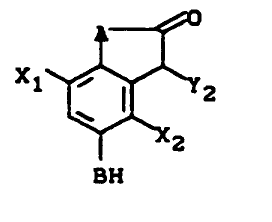 worin A, B, X₁, X₂ und Y₂ die obengenannte Bedeutung haben, mit Verbindungen der Formel 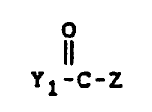 worin
Y₁ und Z die obengenannte Bedeutung haben,
in Gegenwart saurer Kondensationsmittel umsetzt.

2. Verfahren nach Anspruch 1, dadurch gekennzeichnet, daß man die Umsetzung bei 50 - 120° C durchführt.

3. Verfahren nach Anspruch 1, dadurch gekennzeichnet, daß man die Umsetzung als Eintopfreaktion durchführt, indem man zunächst in an sich bekannter Weise Verbindungen der Formel 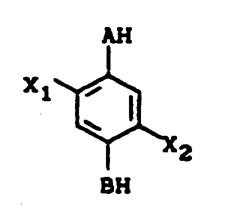
worin
A, B, X₁ und X₂ die obengenannte Bedeutung haben, mit Verbindungen der Formel 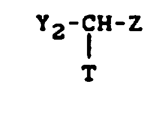 worin
Y₂ und Z obengenannte Bedeutung haben und
T für OH, O-Acyl oder Halogen steht,
mischt und das Umsetzungsprodukt ohne Zwischenmischung mit Verbindungen der Formel 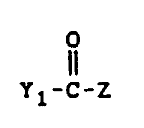 worin
Y₁ und Z die obengenannte Bedeutung haben, sauer kondensiert.

## Claims

1. Process for the preparation of compounds of the formula 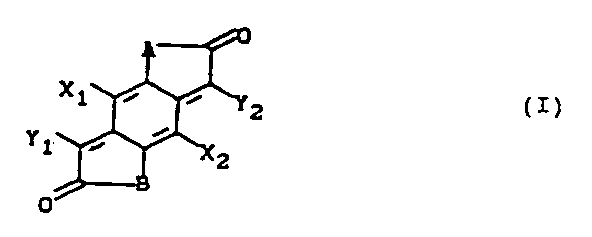 wherein
A and B denote O, S or NQ,
X₁ and X₂ denote hydrogen, halogen, alkyl, alkoxy, aryl or Z and
Y₁ and Y₂ denote alkyl, cycloalkyl, aryl or hetaryl, the radicals mentioned being identical or different, and
Q represents V or acyl,
Z represents COOV, CN or CONVW,
V represents hydrogen, alkyl or aryl and
W represents V,
characterised in that compounds of the formula 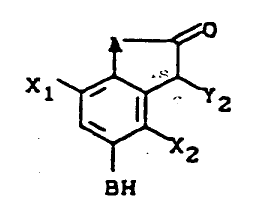 wherein
A, B, X₁, X₂ and Y₂ have the abovementioned meaning, are reacted with compounds of the formula 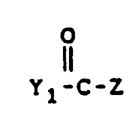 wherein
Y₁ and Z have the abovementioned meaning, in the presence of acid condensing agents.

2. Process according to Claim 1, characterised in that the reaction is carried out at 50-120°C.

3. Process according to Claim 1, characterised in that the reaction is carried out as a one-pot reaction by a procedure in which compounds of the formula 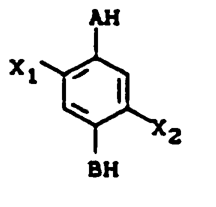
wherein
A, B, X₁ and X₂ have the abovementioned meaning, are first mixed in a manner known per se with compounds of the formula 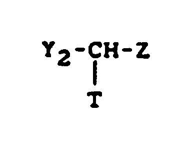
wherein
Y₂ and Z have the abovementioned meaning and
T represents OH, O-acyl or halogen,
and the reaction product is then subjected to acid condensation, without intermediate mixing, with compounds of the formula wherein 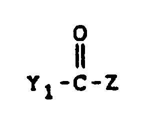 Y₁ and Z have the abovementioned meaning.

## Revendications

1. Procédé de production de composés de formule 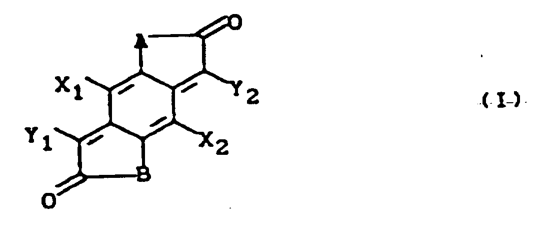 dans laquelle
A et B représentent O, S ou NQ,
X₁ et X₂ représentent de l'hydrogène, un halogène, un groupe alkyle, alkoxy, aryle ou Z et
Y₁ et Y₂ désignent un reste alkyle, cycloalkyle, aryle ou hétaryle, les restes mentionnés étant identiques ou différents et
Q représente V ou un groupe acyle
Z est un groupe COOV, CN ou CONVW,
V est l'hydrogène, un groupe alkyle ou aryle et
W représente V,
caractérisé en ce qu'on fait réagir des composés de formule 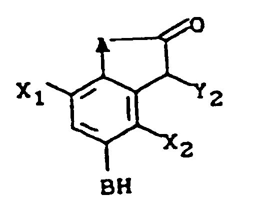 dans laquelle
A, B, X₁, X₂ et Y₂ ont la définition indiquée ci-dessus, avec des composés de formule 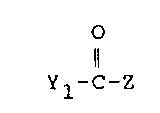 dans laquelle
Y₁ et Z ont la définition indiquée ci-dessus,
en présence d'agents acides de condensation.

2. Procédé suivant la revendication 1, caractérisé en ce qu'on conduit la réaction à 50-120°C.

3. Procédé suivant la revendication 1, caracterisé en ce qu'on conduit la réaction comme réaction en récipient unique en mélangeant tout d'abord d'une manière connue des composés de formule 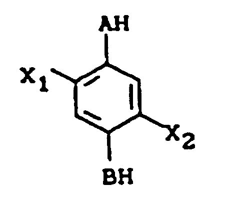 dans laquelle
A, B, X₁ et X₂ ont la définition indiquée ci-dessus, avec des composés de formule 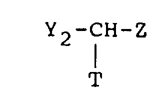 dans laquelle
Y₂ et Z ont la définition indiquée ci-dessus et
T est un groupe OH, O-acyle ou un halogène,
et on soumet le produit de réaction à une condensation acide sans mélange intermédiaire avec des composés de formule 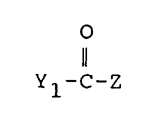 dans laquelle
Y₁ et Z ont la définition indiquée ci-dessus.
